# EUROPÄISCHE PATENTSCHRIFT

(11) **EP 2 422 748 B1**
(45) Veröffentlichungstag und Bekanntmachung des Hinweises auf die Patenterteilung: **27.01.2016**
(21) Anmeldenummer: 11176432.0
(22) Anmeldetag: 03.08.2011
(51) Int. Cl.: A61F 2/24

(54) **Medizinisches Implantat, insbesondere Klappenimplantat, zur Implantation in einen tierischen und/oder menschlichen Körper sowie Verfahren, insbesondere Herstellungsverfahren, zur Herstellung einer Implantationsvorrichtung für das medizinische Implantat**
Medical implant, particularly valve implant, for implantation in an animal and/or human body and method, particularly production method, for producing an implantation apparatus for the medical implant
Implant médical, notamment implant à clapet pour l'implantation dans un corps animal et/ou humain et procédé, notamment procédé de fabrication, pour la fabrication d'un dispositif d'implantation pour l'implant médical

(30) Priorität: 31.08.2010 US 378422 P
(43) Veröffentlichungstag der Anmeldung: 29.02.2012
(73) Patentinhaber: Biotronik AG, 8180 Bülach (CH)
(72) Erfinder: Borck, Alexander, 91086 Aurachtal (DE)
(74) Vertreter: Galander, Marcus

(56) Entgegenhaltungen:
- WO-A1-2008/100599
- WO-A1-2009/144463
- WO-A2-2010/104638
- US-A1- 2003 105 516
- US-A1- 2005 267 560
- US-A1- 2006 287 717
- US-A1- 2009 125 098

## Beschreibung

Die Erfindung betrifft ein medizinisches Klappenimplantat, zur Implantation in einen tierischen und/oder menschlichen Körper nach dem Oberbegriff des Patentanspruchs 1.

In der Medizin kommen Implantate zum Einsatz, die zur Erfüllung von Ersatzfunktionen permanent oder zumindest für einen längeren Zeitraum in einen tierischen und/oder menschlichen Körper eingebracht werden. Bekannt sind z.B. Klappenimplantate, wie etwa Aortenklappenimplantate, welche die Funktion der natürlichen Aortenklappe übernehmen. Hierbei wird das Klappenimplantat nach Expansion der Implantatstruktur sofort nach der Implantation fixiert und nimmt die Position der natürlichen Aortenklappe ein.

Ein häufiges Problem ist hierbei, dass das Implantat mit einer Fehlstellung fixiert wird, was zu einem Versagen des Implantats führen kann. Dies tritt beispielsweise oft bei Kalzifizierung, d.h. die Ablagerung von Kalziumsalzen, insbesondere Kalziumphosphat (Hydroxyapatit) an den Strukturen des Herzens und insbesondere bei stark asymmetrisch kalzifizierte Aortenstenose auf.
Der Erfindung liegt die Aufgabe zugrunde, ein medizinisches Implantat zu schaffen, das exakt und zuverlässig an einer Implantationsstelle implantiert werden kann.

Die Aufgabe wird erfindungsgemäß durch die Merkmale des Patentanspruchs 1 gelöst. Günstige Ausgestaltungen und Vorteile der Erfindung ergeben sich aus den weiteren Ansprüchen, der Zeichnung und der Beschreibung.

Aus dem Stand der Technik bekannt ist US 2003/0105516 A1. Hierbei wird ein Stent beschrieben, der ein erstes ballonexpandierbares Segment besitzt, ein zweites ballonexpandierbares Segment und dazwischen ein selbstexpandierbares Segment.

US 2008/0262593 A1 beschreibt einen Multi-layer Stent.

Die Erfindung geht aus von einem medizinischen Implantat, insbesondere einem Klappenimplantat, zur Implantation im tierischen und/oder menschlichen Körper mit einem wenigstens bereichsweise expandierbaren Grundkörper.

Es wird vorgeschlagen, dass der wenigstens bereichsweise expandierbare Grundkörper einen ersten aktiv expandierbaren Bereich und zumindest einen zweiten passiv expandierbaren Bereich aufweist. Durch die erfindungsgemäße Ausgestaltung kann ein Implantat bereitgestellt werden, das optimal positioniert und gute verankert werden kann. Ferner kann es vorteilhaft auf die Parameter bzw. an anatomischen Gegebenheiten einer Implantationsstelle, wie eine Kalzifizierung einer Blutgefäßwand und/oder eines Annulus, und/oder eine andere, angeborenen und/oder krankhafte Anomalie der Implantationsstelle angepasst werden. Ferner kann ein Druckgradient eines auf das medizinische Implantat wirkenden Fließmediums, wie beispielsweise Blut, homogen gehalten werden, was vorteilhaft zu einer geringen Materialbelastung des Grundkörpers des Implantats und dadurch zu einem geringen Ermüdungsrisiko, insbesondere bei Nitinol Grundkörpern, durch gleichmässiges Öffnen des Grundkörpers führt. Dies wiederum resultiert in eine lange Lebensdauer der Segel und damit der Klappe. Des Weiteren können durch die bessere Funktionalität der Klappe, die so einem höheren Druckgradienten standhalten kann, bei asymmetrisch kalzifiziertem Annulus bessere klinische Ergebnisse gegenüber den herkömmlichen Klappenimplantaten erzielt werden. Durch die erfindungsgemäe Ausgestaltung kann ferner eine Symmetrie in der Flussdynamik des Fließmediums erhöht werden, was das weitere Kalzifizierungsrisiko vorteihaft verringert. Ferner soll Grundkörper zumindest zwei zweite Bereiche aufweisen, sowie das Implantat eine Klappe aufweisen, die in einer axialen Richtung zumindest zwischen zwei zweiten Bereichen angeordnet ist, wobei zumindest ein zweiter Bereich in radialer Richtung weiter außen angeordnet als der erste Bereich.

In diesem Zusammenhang soll unter einem "Implantat" insbesondere ein Körper verstanden werden, der zumindest eine Ersatzfunktion permanent oder für einen längeren Zeitraum bei Implantation in einen tierischen und/oder menschlichen Körper erfüllt. Denkbar wären hier sämtliche, dem Fachmann als zweckdienlich erscheinenden medizinischen Implantate, wie beispielsweise ein Herzschrittmacher, ein Hirnschrittmacher, ein Herzimplantat, ein Cochleaimplantat, ein Retina Implantat, ein zahnmedizinisches Implantat, ein Implantat zum Gelenkersatz, eine Gefäßprothesen oder besonders vorteilhaft wird eine Ausbildung des medizinischen Implantats als ein Klappenimplantat vorgeschlagen. Unter einem "Klappenimplantat" soll insbesondere ein Körper verstanden werden, der zumindest eine Ersatzfunktion eines Rückschlagventils, permanent oder für einen längeren Zeitraum bei Implantation erfüllt. Denkbar wären hier sämtliche, dem Fachmann als zweckdienlich erscheinende medizinische Klappenimplantate, wie beispielsweise ein Aortenklappen-, ein Pulmonalklappen-, ein Mitralklappen- oder ein Trikuspidalklappenimplantat, oder besonders vorteilhaft wird eine Ausbildung des medizinischen Implantats als ein Stent, insbesondere ein Koronarstent, mit einer mit dem Stent reversibel oder irreversibel verbundenen Implantatstruktur vorgeschlagen. In diesem Zusammenhang soll unter einer "Implantatstruktur" insbesondere eine Aortenklappe, eine Pulmonalklappe, eine Mitralklappe oder eine Trikuspidalklappe aus natürlichem und/oder künstlichem Material verstanden werden. Generell wäre jedoch jede andere, dem Fachmann für sinnvoll erscheinende Implantatstruktur denkbar. Durch die Ausführung des Implantats als Stent bzw. dadurch dass der Grundkörper einen Stent umfasst, kann eine konstruktiv einfach zu implantierende Struktur bereitgestellt werden.

Ferner soll in diesem Zusammenhang unter einem "Grundkörper" insbesondere eine Struktur, wie beispielsweise ein Drahtgeflecht, verstanden werden, die im Wesentlichen eine Gestalt und/oder eine Form des Klappenimplantats bzw. insbesondere eine Form des Stents und/oder den Stent selbst bildet. Zudem ist der Grundkörper vorzugsweise aus einem elastischen oder superelastischen Material, etwa einem metallischen Material und/oder aus einer Kombination von mehreren metallischen Materialien gefertigt, wie beispielsweise Eisen, Magnesium, Nickel, Wolfram, Titan, Zirkonium, Niob, Tantal, Zink, Silizium, Lithium, Natrium, Kalium, Kalzium, Mangan und/oder jedem anderen, dem Fachmann als sinnvoll erscheinenden Material. Möglich wäre auch eine Zink-Kalziumlegierung. Unter der Wendung "aktiv expandierbar" soll insbesondere verstanden werden, dass der Bereich selbständig bzw. selbsttätig, also ohne fremde Hilfe expandiert bzw. expandierbar ist. Vorteilhafterweise ist der aktiv expandierbare Bereich aus einem Formgedächtnis-Material, wie beispielsweise eine Kupfer-Zink-Aluminium-Legierung und/oder Nickel-TitanLegierung, vorzugsweise Nitinol, gefertigt. Zudem wäre es auch denkbar, dass zwei aktiv expandierbare Bereiche vorgesehen sind, die gleiche oder unterschiedliche Expansionscharakteristika aufweisen. Unter "passiv expandierbar" soll insbesondere verstanden werden, dass der Bereich unselbständig und/oder mittels einer von außen zugeführten Kraft expandierbar bzw. plastisch verformbar ist. Eine Expansion mit Hilfe eines selbstexpandierbaren Materials und/oder durch den ersten Bereich soll hier insbesondere nicht verstanden werden. Konstruktiv einfach kann die passive Expansion mittels eines Ballonkatheters erfolgen. Ferner kann es vorteilhaft sein, wenn der passiv expandierbare Bereich bzw. der Grundkörper zumindest Kobalt und/oder Chrom aufweist, vorzugsweise in der Form von Edelstahl bzw. medizinischem Edelstahl und/oder eines Cr-Ni-Fe-Stahl - hier bevorzugt die Legierung 316L - oder einem Co-Cr-Stahl. Durch diese Ausgestaltung kann ein Implantat bereitgestellt werden, das zu zufrieden stellenden Beschichtungsergebnissen führt sowie eine gute Dilatierbarkeit und eine vorteilhafte Flexibilität gepaart mit einer hohen Stabilität aufweist. Grundsätzlich wäre es jedoch auch denkbar, dass der Grundkörper des Klappenimplantats mindestens teilweise aus Kunststoff, einer Keramik und/oder aus einem biodegradierbaren Material besteht. Mittels der beiden ungleich expandierbaren Bereiche können vorteilhaft unterschiedliche Expansionsmechanismen angewendet werden, was zu einem besonders vielseitig einsetzbaren Implantat führt.

Des Weiteren wird vorgeschlagen, dass der zweite Bereich zeitlich nach dem ersten Bereich expandierbar ist. Unter der Wendung "zeitlich nach" soll insbesondere nachträglich und/oder ein Zeitversatz von mehr als 10 Sekunden, bevorzugt vom mehr als 60 Sekunden und besonders vorteilhaft von mehr als 300 Sekunden zwischen der aktiven und der passiven Expansion verstanden werden. Durch die Realisierung des Zeitversatzes kann eine Positionierung verfeinert werden, wodurch vorteilhaft Fehlstellungen des Implantats nach der Vorimplantation durch den aktiv expandierbaren Bereich mittels des passiven Bereichs ausgeglichen werden können. Hierdurch kann konstruktiv einfach ein Versagen des Implantats verhindert werden.

Vorteilhafterweise ist der erste Bereich dazu vorgesehen, bei einer Expansion zumindest des ersten Bereichs den zweiten Bereich in einer transversalen Richtung aufzuweiten. In diesem Zusammenhang soll unter einer "transversalen Richtung" insbesondere eine Richtung verstanden werden, die, ausgehend von einem geometrischen Schwerpunkt einer von dem ersten Bereich fiktiv aufgespannten Fläche, nach Außen weist. Die transversale Richtung ist insbesondere eine radiale Richtung und ein "geometrischer Schwerpunkt der Fläche" meint insbesondere einen Mittelpunkt und besonders vorteilhaft einen Kreismittelpunkt einer Fläche, insbesondere einer Kreisfläche, mit einer maximalen Erstreckung im implantierten Zustand des Implantats im Wesentlichen senkrecht zur Fließrichtung des Fließmediums. Dadurch ist die Implantatstruktur bzw. die Klappe passgenau in einen Innenquerschnitt des Implantats aufgenommen bzw. aufnehmbar. Der Begriff "Aufweiten" definiert hier insbesondere eine Vergrößerung eines Querschnitts einer fiktiven Fläche, die der zweite Bereich aufspannt. Durch die Aufweitung kann im vorimplantierten Zustand des Implantats effektiv eine Behinderung eines Blutflusses durch den zweiten Bereich verhindert werden.

Hierbei können der erste aktiv expandierbare Bereich und der zweite passiv expandierbare Bereich in jeder beliebigen, dem Fachmann für zweckdienlich erscheinenden Anordnung zueinander, wie beispielsweise hintereinander und/oder in transversaler Richtung übereinander, ausgestaltet sein. Zweckmäßigerweise weist zumindest ein Anteil des zweiten Bereichs gegenüber dem ersten Bereich, ausgehend von dem geometrischen Schwerpunkt bzw. Mittelpunkt der von dem ersten Bereich aufgespannten fiktiven Fläche, einen größeren Abstand zu dem Schwerpunkt/Mittelpunkt auf als ein Abstand des ersten Bereichs zu dem Schwerpunkt/Mittelpunkt bzw. ist erfindungsgemäß der zweite Bereich in radialer Richtung weiter außen angeordnet als der erste Bereich und/oder der zweite Bereich ist über dem ersten Bereich angeordnet. Ferner ist der zweite Bereich in Umfangsrichtung um den ersten aktiv expandierbaren Bereich angeordnet. Des Weiteren erstreckt sich der zweite Bereich bevorzugt um den gesamten Umfang des ersten Bereichs. Grundsätzlich wäre jedoch auch eine Erstreckung über Abschnitte des Umfangs denkbar.

Eine konstruktiv einfache Aufweitung des zweiten Bereichs kann erreicht werden, wenn der zweite Bereich mit dem ersten Bereich über einen sich in einer axialen Richtung erstreckenden Bereich verflochten ist. Hierbei soll unter dem Begriff "Verflochten" insbesondere verwoben, verstrickt, verschlungen, verkettet, verknotet, gewirkt und jedes andere, dem Fachmann für sinnvolle erscheinendes Synonym verstanden werden. Die Verbindung zwischen den beiden Bereichen ist vorteilhaft flexibel bzw. nicht starr, sodass eine Relativbewegung in axialer Richtung und/oder in Umfangsrichtung zwischen den beiden Bereichen erfolgen kann.

Ferner ist es vorteilhaft, wenn der aktiv expandierbare Bereich bevorzugt aus Zellen aufgebaut ist. Diese Zellen können jede, dem Fachmann als zweckdienlich erscheinende Form, wie rund, oval, dreieckig, rechteckig und/oder Rautenform aufweisen. Diese Form ist insbesondere dazu ausgebildet, faltbar zu sein. Besonders bevorzugt sind die Zellen im Wesentlichen rautenförmig ausgestaltet. Unter der Wendung "im Wesentlichen rautenförmig" soll hier insbesondere verstanden werden, dass auch Formen, die einer Raute bzw. einem Rhombus ähnlich sind, wie beispielsweise eine rautenähnliche Form mit abgerundeten Ecken und/oder konkaven und/oder konvexen Seiten unter dem Begriff "rautenförmig" verstanden werden sollen. Ferner kann auch der zweite passiv expandierbare Bereich aus Zellen mit einer und/oder mehreren obengenannten Formen und Eigenschaft aufgebaut sein. Durch diese Form kann ein besonders stabiles Grundgerüst des Implantats bereitgestellt werden.

In einer weiteren Ausgestaltung der Erfindung wird vorgeschlagen, dass der Grundkörper mittels einer Expansion in seiner axialen Länge verkürzbar ist. Dies kann beispielsweise mittels der rautenförmigen Zellen konstruktiv besonders einfach erfolgen. Durch die erfindungsgemäße Ausgestaltung kann vor der Expansion eine gegenüber unverkürzbaren Implantaten größere Länge des Implantats vorteilhaft zur Unterbringung einer Implantatstruktur bzw. einer Klappe genutzt werden. Ferner kann so bei der Herstellung einer Implantatvorrichtung mit dem erfindungsgemäßen Implantat ein Krimpradius vorteilhaft minimiert werden.

Ferner wird vorgeschlagen, dass der zweite Bereich dazu vorgesehen ist, einen Unterschied in einer Form des Innenquerschnitts des Grundkörpers und einer Querschnittsfläche der Implantationsstelle auszugleichen. Unter "vorgesehen" soll insbesondere speziell ausgestattet, ausgelegt und/oder vorbereitet verstanden werden. In diesem Zusammenhang soll unter einer "Form des Innenquerschnitts des Grundkörpers" insbesondere eine weitestgehend runde bzw. zylinderförmige Form verstanden werden, so dass sich Segel der Klappe komplikationslos Öffnen und Schließen können. Unter einer "Querschnittsfläche einer Implantationsstelle" soll hier insbesondere eine stark asymmetrisch bzw. unrunde Stelle insbesondere mit einer kalzifizierten Aortenstenose verstanden werden. Der zweite Bereich passt somit die uneinheitlichen Formen des Außendurchmessers der Klappe bzw. des Innenquerschnitts des Grundkörpers vorteilhaft an die Querschnittsfläche der Implantationsstelle an, wodurch das Implantat in besonderem Maße die örtlichen Gegebenheiten an der Implantationsstelle berücksichtigt. Dadurch kann vorteilhaft eine Unsymmetrie der Blutgefäßwand bzw. des Annulus ausgeglichen werden und dennoch eine weitestgehend runde, symmetrische Innenform des Grundkörpers zur benötigten fehlerfreien und komplikationslosen Funktion von Segeln der Klappe beibehalten werden.

Des Weiteren kann es vorteilhaft sein, wenn das medizinische Implantat ein Trennmittel aufweist, das den ersten Bereich von dem zweiten Bereich trennt. In diesem Zusammenhang soll unter einem "Trennmittel" insbesondere jedes dem Fachmann für zweckdienlich erscheinendes Mittel, wie ein Abstandshalter und/oder insbesondere eine Beschichtung verstanden werden, wobei unter einer "Beschichtung" insbesondere eine zumindest teilweise und bevorzugt eine vollständige Ummantelung des ersten und/oder des zweiten Bereichs bzw. deren Verstrebungen bzw. Stentstruts verstanden werden soll. Besonders vorteilhaft ist die Beschichtung von einem amorphen Siliziumcarbid gebildet. Generell wäre jedoch jede andere, dem Fachmann für einsetzbar erscheinende Beschichtung aus isolierenden oder Halbleitermaterialien denkbar, die ein Inkontaktbringen der Materialien der beiden Bereiche und insbesondere der metallischen Materialien, wie insbesondere NiTi oder CoCr, in Gegenwart von Elektrolyten wirkungsvoll verhindert. Durch die Beschichtung kann konstruktiv einfach und Platz sparend ein Kontaktproblem der unterschiedliche edlen Metalle der beiden Bereiche gelöst werden.

Zudem wird vorgeschlagen, dass das Implantat zumindest ein Verankerungsmittel aufweist, wodurch das Implantat besonders komfortabel fixiert werden kann. Besonders vorteilhaft ist das Verankerungsmittel am zweiten Bereich angeordnet und weist im bestimmungsgemäßen Endzustand bzw. im implantierten Zustand zumindest einen Extremalpunkt auf, der ausgehend von dem geometrischen Schwerpunkt/Mittelpunkt der von dem ersten Bereich aufgespannten Fläche einen größeren Abstand zu dem Schwerpunkt/Mittelpunkt aufweist als ein Abstand des ersten Bereichs zu dem Schwerpunkt/Mittelpunkt. In diesem Zusammenhang soll unter einem "Verankerungsmittel" insbesondere eine Schlaufe, ein Haken, eine Spitze und/oder ein anderes, dem Fachmann für anwendbar erachtetes Mittel verstanden werden. Unter einem "bestimmungsgemäßen Endzustand" soll hier insbesondere ein implantierter Zustand des Implantats an der Implantationsstelle, wie beispielsweise einer Stelle einer defekten Herzklappe und/oder einem Annulus verstanden werden. Hierbei ist das Implantat bzw. der Stent mit den zwei Bereichen expandiert und an der Implantationsstelle in der korrekten Position verankert. Ferner soll unter dem Begriff "Extremalpunkt" insbesondere ein Maximum in der Erstreckung eines Außenquerschnitts, ausgehend vom geometrischen Mittelpunkt des Grundkörpers nach außen und im implantierten Zustand in Richtung einer Wand eines Blutgefäßes, verstanden werden. Durch die Realisierung des Extremalpunkts und den größeren Abstand kann da Implantat vorteilhaft an vorbestimmten Punkten fixiert werden.

Das Verankerungsmittel ist zudem besonders vorteilhaft unabhängig von dem restlichen zweiten Bereich bewegbar, wobei unter "bewegbar" hier insbesondere radial bewegbar und besonders vorteilhaft radial bewegbar in Richtung der Wand des Blutgefäßes, wie beispielsweise der Aortenwand, während der Expansion mittels eines Expansionsbereichs eines Expansionmittels, wie bspw. eines Ballonkatheters, verstanden werde soll. Bevorzugt weist das Verankerungsmittel eine geringere Rückhaltekraft auf, als der restliche zweite Bereich, wobei unter "Rückhaltekraft" hier insbesondere eine Kaft verstanden werden soll, deren Vektor in Richtung des geometrischen Schwerpunkts bzw. dem Kreismittelpunkt des Grundkörpers weist. Die geringere Rückhaltekraft ist beispielsweise bedingt durch eine Verminderung von Kontaktstellen des Verankerungsmittels mit dem restlichen zweiten Bereich gegenüber Strukturen bzw. Zellen des zweiten Bereichs untereinander. Durch die geringere Rückhaltekraft weist das Verankerungsmittel bei der Expansion mittels des Expansionsbereichs ein größeres Vermögen als der restliche zweite Bereich auf, radial nach Außen d. h. in Richtung der Wand des Blutgefäßes bewegt zu werden. Hierbei ist dieser Rückhaltekräfteunterschied unabhängig von einem von Außen einwirkenden Widerstand, wie beispielsweise der Kraft der Wand des Blutgefäßes (Aorta). Folglich ist das Verankerungsmittels relativ zu einer Fließrichtung des Fließmediums schräg anordenbar. Mittels des Verankerungsmittels kann vorteilhaft eine variable Außenkontur des Stents im implantierten Zustand eingestellt werden. Zudem kann eine gute Verankerung bei vorteilhaft geringem Kraftaufwand durch das Expansionsmittel bereitgestellt werden.

Eine besonders exakte Positionierung und Fixierung kann vorteilhaft erreicht werden, wenn der Grundkörper erfindungsgemäß zumindest zwei zweite Bereiche aufweist. Grundsätzlich kann das Implantat auch beliebig viele zweite passiv expandierbare Bereiche aufweisen. Bei der Ausführung mit zwei zweiten Bereichen wird als Expansionsmittel vorteilhaft ein Doppelballonkatheter und bei mehr als zwei zweiten Bereichen ein segmentierter Ballonkatheter verwendet. Bevorzugt ist zumindest je ein erster Bereich axial zwischen zwei zweiten Bereichen angeordnet.

Eine bevorzugte Weiterbildung besteht darin, dass das Implantat eine Klappe aufweist, die in einer axialen Richtung zumindest zwischen zwei zweiten Bereichen bzw. zwischen zwei nachträglich aufweitbaren angeordnet ist. Hierbei kann die Klappe mittels jeder dem Fachmann für sinnvoll erachteten Verbindungsart, wie beispielsweise Nähen und/oder Kleben mit dem Grundkörper bzw. mit dem ersten Bereich verbunden sein. Durch diese Anordnung wird ein Implantat bereitgestellt, das im implantierten Zustand eine vorteilhaft taillierte Form einnehmen kann, die für eine Selbstfindung / Selbstpositionierung der optimalen Position von Vorteil ist.

Eine weitere Ausgestaltung der Erfindung sieht vor, dass die zumindest zwei zweiten Bereiche im bestimmungsgemäßen Endzustand in Fließrichtung des Fließmediums axial vor und hinter einem Annulus angeordnet sind. Unter einer "Fließrichtung eines Fließmediums" soll hier insbesondere die wissenschaftlich bekannte Fließrichtung von arteriellem und/oder venösem Blut im Herzen und besonders vorteilhaft im Falle der Aortenklappe der Fluss von Blut vom linken Ventrikel in die Aorta verstanden werden. Hierbei handelt es sich bei dem Annulus bevorzugt um den Aortenannulus. Durch die Realisierung der erfindungsgemäßen Anordnung kann das Implantat besonders gut an die Anatomie des Herzens bzw. einer Herzklappenregion mit beispielsweise einem Aortenbulbus angepasst werden, insbesondere im Fall der taillierten Form.
Besonders vorteilhaft ist eine Ausgestaltung des medizinischen Implantats als Aortenklappe, wodurch eine ausgefeilte Ersatzstruktur für die am häufigsten mit Fehlfunktionen behaftete Herzklappe bereitgestellt werden kann. Auch können Komplikationen wie z.B. Störungen der Mitralklappe oder eine Notwendigkeit eines Herzschrittmachers günstigerweise reduziert werden. Ebenso ist eine Ausgestaltung als Pulmonalklappe oder auch eine Ausgestaltung als Mitralklappe denkbar.

Vorteilhaft kann am Implantat eine ablagerungshemmende, insbesondere kalzifizierungshemmende, Beschichtung vorgesehen sein, insbesondere Homocysteinsäure. Damit kann die Gefahr eine Störung oder ein Funktionsausfall des Klappenimplantats weiter verringert werden.

Die Erfindung geht zudem aus von einem Verfahren, insbesondere einem Herstellungsverfahren, zur Herstellung einer Implantationsvorrichtung für ein medizinisches Implantat mit einem Grundkörper, mit einem ersten aktiv expandierbaren Bereich und einem zweiten passiv expandierbaren Bereich.

Es wird vorgeschlagen, dass der Grundkörper auf ein Expansionsmittel montiert wird, wobei zumindest der zweite Bereich des Grundkörpers in Umfangsrichtung um einen Expansionsbereich des Expansionsmittels angeordnet wird und der resultierende Implantationskomplex mit einem Rückhaltemittel kombiniert wird. In diesem Zusammenhang soll unter einer "Implantationsvorrichtung" insbesondere eine Vorrichtung verstanden werden, mittels der eine Implantation durchgeführt wird und die das Implantat aufweist. Unter einem "Implantationskomplex" soll insbesondere ein Gebilde aus dem Implantat und einem weiteren Teil, wie beispielsweise das Expansionsmittel bzw. der Ballonkatheter, verstanden werden. Ein "Rückhaltemittel" stellt hier insbesondere ein Mittel, wie beispielsweise ein dem Fachmann bekannter Überschlauch, dar, das eine aktive Expansion des ersten Bereichs verhindert. Eine Verbindung des Implantats mit dem Expansionsmittel erfolgt bevorzugt mittels einer reversiblen Verbindungsart, wobei jede dem Fachmann für passend erscheinende Verbindungsart, wie bspw. Crimpen, in Frage kommt. Durch die erfindungsgemäße Ausgestaltung kann eine Implantationsvorrichtung bereitgestellt werden, die vorteilhaft zwei Expansionsmechanismen kombiniert.
Mittels des oben beschriebenen Implantats und der beschriebenen Implantationsvorrichtung kann ein Verfahren, insbesondere ein Implantationsverfahren, zur Implantation des medizinischen Implantats mittels der Implantationsvorrichtung in einem tierischen und/oder menschlichen Körper durchgeführt werden, bei dem in einem ersten Schritt der Implantationskomplex aus dem Rückhaltemittel zur Expansion des ersten Bereichs des Grundkörpers des Implantats und zur Vorpositionierung des Implantats entlassen wird und in einem zweiten Schritt der zweiter Bereich des Grundkörpers des Implantats mittels des Expansionsbereich des Expansionsmittels zur endgültigen Positionierung und Verankerung an der Implantationsstelle expandiert wird.

Die Erfindung ist nachfolgend beispielhaft, anhand von einem in Zeichnungen dargestelltes Ausführungsbeispiel näher erläutert. Es zeigen:
- Fig. 1: ein Ausschnitt eines nicht zur Erfindung gehörenden medizinisches Implantats mit einem ersten Bereich und einem zweiten Bereich in einer perspektivischen Ansicht,
- Fig. 2a: eine Zelle des Implantats der Fig. 1 im zusammengefalteten Zustand,
- Fig. 2b: die Zelle der Fig. 2a im expandierten Zustand,
- Fig. 3: das Implantat der Figur 1 in einer schematischen Darstellung mit zwei passive expandierbaren Bereichen,
- Fig. 4: das Implantat der Fig. 3 im implantierten Zustand in einer Aorta,
- Fig. 5: einen Schnitt IV-IV durch die Aortenwand mit implantiertem Implantat gemäß der Fig. 4,
- Fig. 6: eine schematische Darstellung einer Herstellung einer Implantationsvorrichtung mit einem erfindungsgemäßen Implantat und
- Fig. 7: eine schematische Darstellung des Einführens der Implantatvorrichtung gemäß der Fig. 6 an einer Implantationsstelle.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Zur Vermeidung unnötiger Wiederholungen wird bei nicht näher beschriebenen Elementen in einer Figur auf die jeweilige Beschreibung der Elemente in vorstehenden Figuren verweisen.

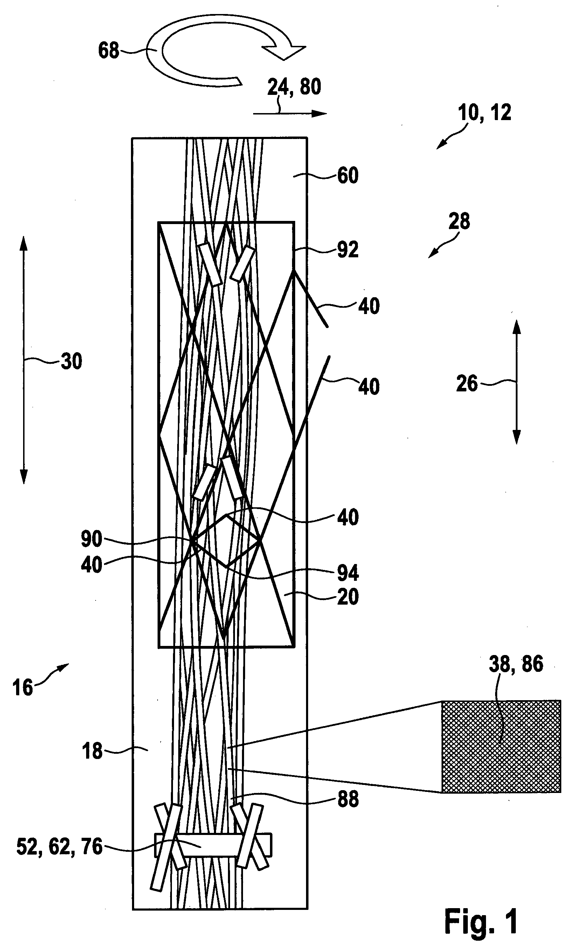

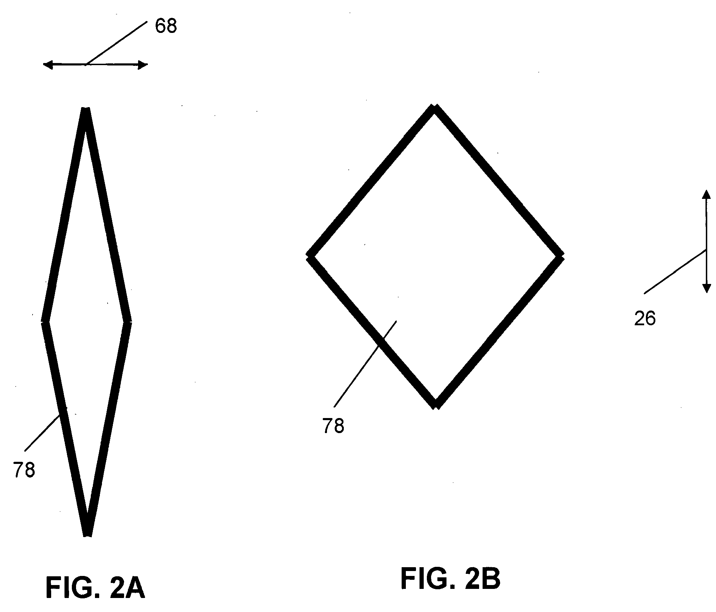

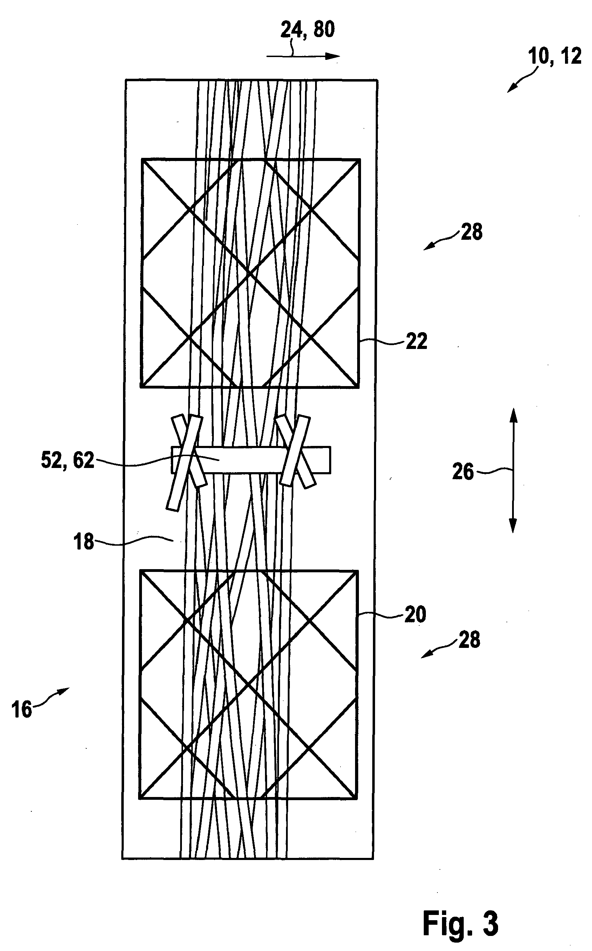

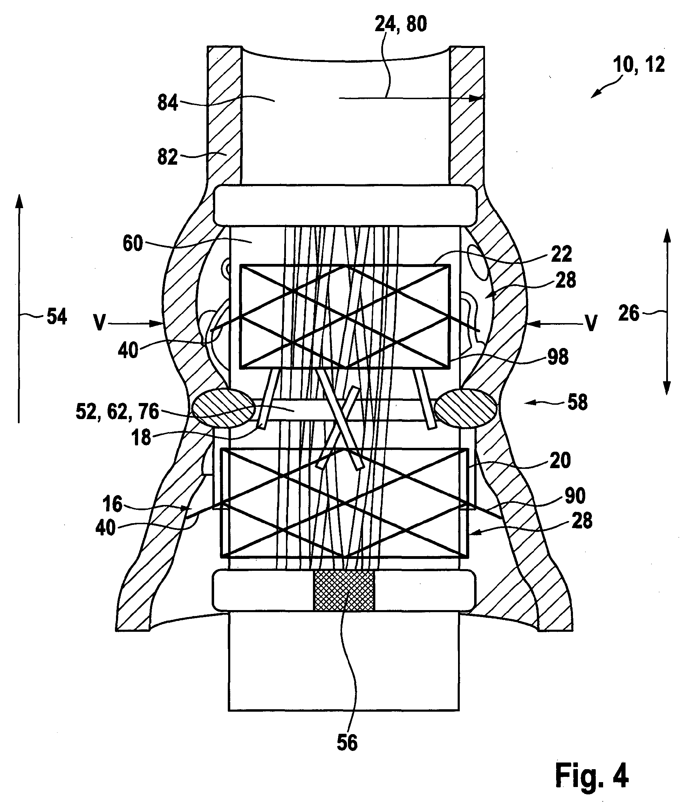

Fig. 1 zeigt einen Teil eines medizinischen Implantats 10 bzw. eines Klappenimplantats 12 zur Implantation in einem tierischen und/oder menschlichen Körper 14, mit einem bereichsweise expandierbaren Grundkörper 16, der einen selbstexpandierenden Stent 60 umfasst. Das Implantat 10 ist ferner ein Aortenimplantat, indem eine Implantatstruktur 76, ausgestaltet als künstliche perkutane Aortenklappe 62, an dem Grundkörper 16 bzw. am Stent 60 befestigt ist. Der Grundkörper 16 weist als Grundkörperstruktur ein Drahtgeflecht auf, das von einem ersten aktiv expandierbaren Bereich 18 gebildet wird und aus Nitinol gefertigt ist. Das Drahtgeflecht bzw. der Grundkörper 16 weist eine Vielzahl von Zellen 78 auf, die im Wesentlichen rautenförmig ausgestaltet sind und in Unfangsrichtung 68 nebeneinander und in axialer Richtung 26 hintereinander angeordnet sind. Durch diese Ausgestaltung kann das Implantat 10 gefaltet werden und bei einer Selbstexpansion weiten sich die Zellen 78 in Umfangsrichtung 68 auf, wobei sich ihre Erstreckung in axialer Richtung 26 verkürzt, wie dies exemplarisch für eine Zelle 78 in den Fig. 2a und 2b dargestellt ist. Hierdurch ist auch der Grundkörper 16 mittels der Expansion in seiner axialen Länge 30 verkürzbar, wie dies insbesondere im Vergleich der Darstellung des Implantats 10 in den Fig. 3 und 4 deutlich wird.

Ferner weist der Grundkörper 16 einen zweiten passiv expandierbaren Bereich 20 auf, der aus einem medizinischen Edelstahl gebildet ist, der zumindest Kobalt und/oder Chrom aufweist. Ferner weist auch der zweite Bereich 20 analog zu dem ersten Bereich 18 eine Vielzahl von rautenförmigen Zellen 78 auf. Der zweite Bereich 20 ist mit dem ersten Bereich 18 über einen sich in axialer Richtung 26 erstreckenden Bereich 28 verflochten, wobei die Verflechtung so ausgebildet ist, dass eine Relativbewegung zwischen den Bereichen 18, 20 stattfinden kann. Der Übersichtlichkeit halber ist die Verflechtung der Bereiche 18, 20 nur exemplarisch in der Fig. 1 gezeigt.

Durch diese Anordnung der Bereiche 18, 20 relativ zueinander, ist der erste Bereich 18 dazu vorgesehen, bei seiner Expansion den zweiten Bereich 20 in einer transversalen Richtung 24 bzw. in einer radiale Richtung 80, also im bestimmungsgemäßen Endzustand bzw. im implantierten Zustand des Implantats 10 hin zu einer Wand 82 eines Blutgefäßes 84, wie beispielsweise einer Aorta, aufzuweiten.

Zudem weist das Implantat 10 ein Trennmittel 38 auf, das den ersten Bereich 18 von dem zweiten Bereich 20 trennt. Dieses Trennmittel 38 wird von einer Beschichtung 86 aus einem amorphen Siliziumcarbid gebildet und ist auf den ersten aktiv expandierbaren Bereich 18 des Grundkörpers 16 bzw. auf dessen Stentstruts 88 aufgebracht (symbolisch in Fig. 1 gezeigt).

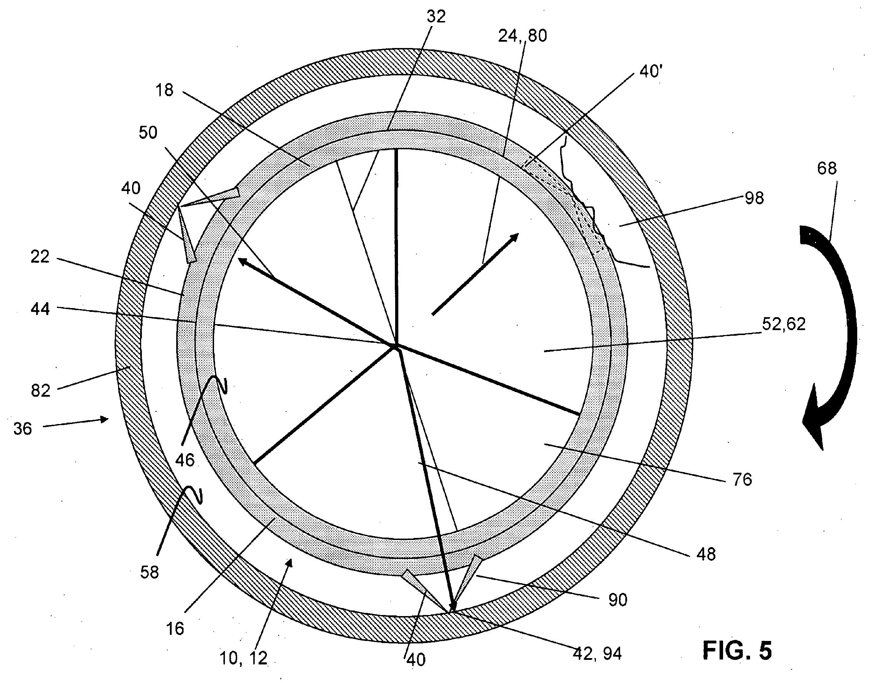

Des Weiteren weist das Implantat 10 bzw. der Grundkörper 16 mehrere Verankerungsmittel 40 am zweiten Bereich 20 auf. Diese Verankerungsmittel 40 sind von winklig angeordneten Stegen 90 gebildet, die somit eine V-Form aufweisen. Ein nicht mit dem anderen Steg 90 verbundenes Ende eines Stegs 90 ist an einem Kreuzungspunkt 92 einer Zelle 78 des zweiten Bereichs 20 angeformt. An einer Verbildungsstelle 94 der Stege 90 bzw. einer Spitze des Vs fehlt dadurch eine Befestigungsstelle mit dem zweiten Bereich 20, wodurch das Verankerungsmittel 40 eine niedrigere Rückhaltekraft aufweist, wie die Zellen 78 des zweiten Bereichs 20. Somit sind die Verankerungsmittel 40 relativ zu dem zweiten Bereich 20 bewegbar bzw. in radialer Richtung 80 bewegbar und relativ zu einer Fließrichtung 54 eines Fließmediums 56, wie beispielsweise Blut, schräg mit einem maximalen Winkel von 45° anordenbar. Wird nun das Implantat 10 bzw. der zweite Bereich 20 mit einem Ballonkatheter aufgeweitet, können die Verankerungsmittel 40 radial weiter nach außen bzw. hin zur Aortenwand gedrückt werden. Somit weist ein Verankerungsmittel 40 im bestimmungsgemäßen Endzustand einen Extremalpunkt 42 an der Verbindungsstelle 94 der Stege 90 auf. Dieser hat, ausgehend von einem geometrischen Schwerpunkt 44 bzw. einem Kreismittelpunkt einer von dem ersten Bereich 18 aufgespannten Fläche 46 einen größeren Abstand 48 zu dem Schwerpunkt 44 als ein Abstand 50 des ersten Bereich 18 zu dem Schwerpunkt 44 (vgl. Fig. 5).

In der Fig. 3 ist das vollständige Implantat 10 gezeigt. Hierbei weist der Grundkörper 16 zwei zweite Bereiche 20, 22 auf, wobei der zweite zweite Bereich 22 konstruktiv und funktional analog zu dem ersten zweiten Bereich 20 ausgeführt ist. Ferner ist eine Klappe 52 in axialen Richtung 26 zwischen den zwei zweiten Bereichen 20, 22 angeordnet.

Fig. 4 zeigt in einer Ansicht schematisch das medizinische Implantat 10 in implantierter Form z.B. in einem Annulus 58 einer natürlichen Aortenklappe, der in dem Blutgefäß 84, bzw. der Aorta, vor einem linken Ventrikel 96 des Herzens angeordnet ist. Hierbei ist der erste zweite Bereich 20 in Fließrichtung 54 vor dem Annulus 58 und der zweite zweite Bereich 20 nach dem Annulus 58 angeordnet.

Wird nun das Implantat 10 implantiert, kommt es mittels der Selbstexpansion des aktiv expandierbaren Bereichs 18 des Grundkörpers 16 zu einer Vorpositionierung des Implantats 10. Hierbei wirken sich insbesondere Anomalien am Annulus 58 oder Kalzifizierungen 98 nicht wesentlich auf die Position des Implantats 10 aus. Die zweiten Bereiche 20, 22 werden nun zeitlich nach dem ersten Bereich 18 expandiert. Hierbei kommt es abhängig von den Gegebenheiten an einer Implantationsstelle 36 zu einer Nachpositionierung bzw. einer Einnahme eines optimalen Sitzes des Implantats 10 und durch einen Presssitz der zweiten Bereiche 20, 22 mit der Wand 82 bzw. durch die Bewegung der Verankerungen 40 in Richtung der Wand 82 des Blutgefäßes 84 zu einer Fixierung des Implantats 10.

Befinden sich nun Kalzifizierungen 98 an der Wand 82 des Blutgefäßes 84 bewegen sich die zweiten Bereich 20, 22 bis sie in Anlage mit der Kalzifizierung 98 kommen. Zudem wird beispielsweise verhindert, dass Verankerungsmittel 40' nach außen gedrängt werden können, wie dies die Fig. 5, die einen Schnitt durch die Aortenwand mit Aufsicht auf das Implantat 10 darstellt, zeigt. Somit ist der zweite Bereich 20 bzw. sind die Verankerungsmittel 40 dazu vorgesehen, einen Unterschied in einer Form eines Innenquerschnitts 32 des Grundkörpers 16 und einer Querschnittsfläche 34 der Implantationsstelle 36 auszugleichen.

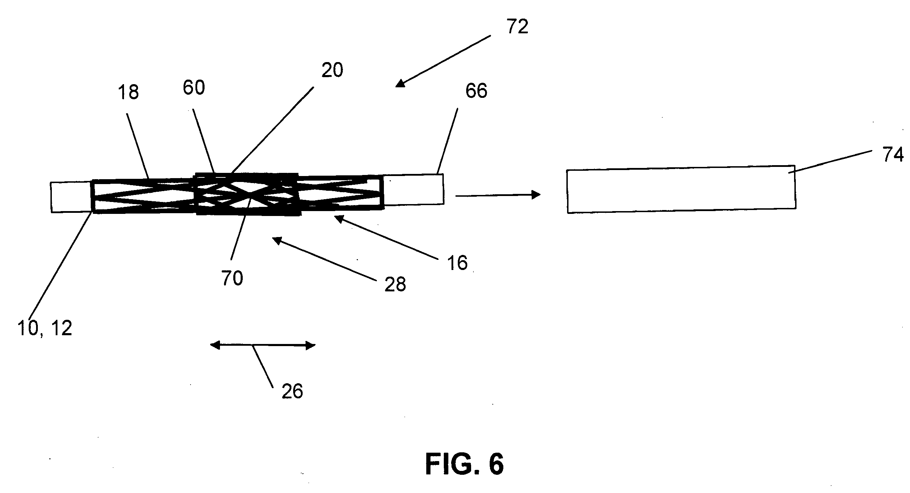

In der Fig. 6 ist schematisch eine Herstellung einer Implantationsvorrichtung 64 für das medizinische Implantat 10 gezeigt. Hierbei wird der Grundkörper 16 auf ein Expansionsmittel 66 montiert bzw. durch einen Crimpprozess bei -78°C befestigt. Dabei wird der zweite Bereich 20 des Grundkörpers 16 in Umfangsrichtung 68 um einen Expansionsbereich 70 des Expansionsmittels 66, wie einen Ballonbereich eines Ballonkatheters, angeordnet (analog für Bereich 22 auf einen Doppelballonkatheter). Danach wird der hieraus resultierende Implantationskomplex 72 aus Implantat 10 und Expansionsmittel 66 mit dem Rückhaltemittel 74 kombiniert bzw. wird der Implantationskomplex 72 in das Rückhaltemittel 74 eingeschoben.

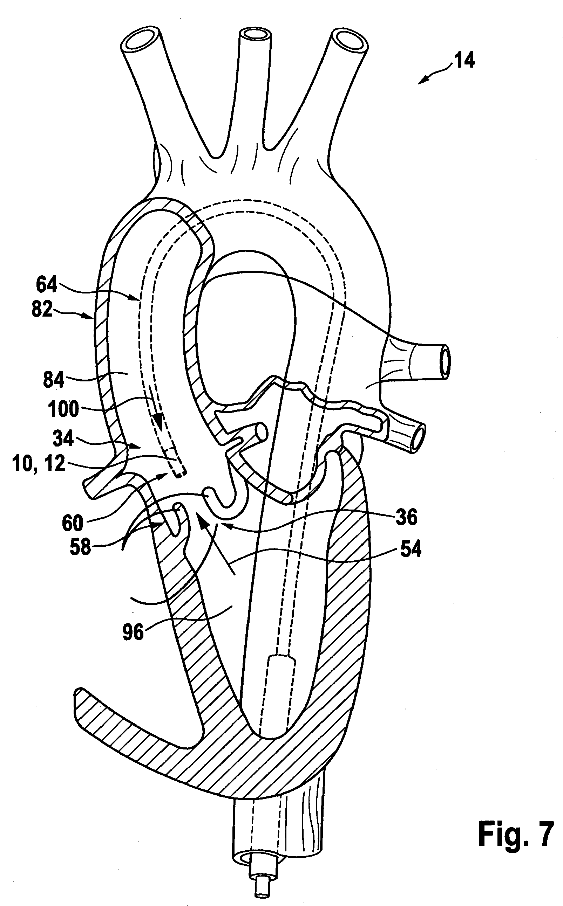

In der Fig. 7 ist das Einführen des medizinischen Implantats 10 schematisch in einer Teilschnittdarstellung illustriert. Der Implantationskomplex 72 aus Ballonkatheter und Implantat 10 wird in an sich bekannter Weise an die Implantationsstelle 36, z.B. den Annulus 58 der natürlichen Aortenklappe mit Segeln zugeführt. Hierbei ist eine Implantationsrichtung 100 entgegengesetzt zur Fließrichtung 54. Ist die korrekte Position erreicht, wird der Implantationskomplex 72 aus dem Rückhaltemittel 74 entlassen, wodurch der erste aktiv expandierbare Bereich 18 selbstexpandiert und das Implantat 10 vorpositioniert. In einem zweiten zeitlich nachfolgenden Schritt, wird der zweite Bereich 20 des Grundkörpers 16 mittels des Expansionsbereichs 64 des Expansionsmittels 60 passiv expandiert. Dies führt nun zur endgültigen Positionierung und Verankerung des Implantats 10 an der Implantationsstelle 36.

### Bezugszeichenliste

- 10: Implantat
- 12: Klappenimplantat
- 14: Körper
- 16: Grundkörper
- 18: Bereich
- 20: Bereich
- 22: Bereich
- 24: Richtung
- 26: Richtung
- 28: Bereich
- 30: Länge
- 32: Innenquerschnitt
- 34: Querschnittsfläche
- 36: Implantationsstelle
- 38: Trennmittel
- 40: Verankerungsmittel
- 42: Extremalpunkt
- 44: Schwerpunkt
- 46: Fläche
- 48: Abstand
- 50: Abstand
- 52: Klappe
- 54: Fließrichtung
- 56: Fließmediums
- 58: Annulus
- 60: Stent
- 62: Aortenklappe
- 64: Implantationsvorrichtung
- 66: Expansionsmittel
- 68: Umfangsrichtung
- 70: Expansionsbereich
- 72: Implantationskomplex
- 74: Rückhaltemittel
- 76: Implantatstruktur
- 78: Zelle
- 80: Richtung
- 82: Wand
- 84: Blutgefäß
- 86: Beschichtung
- 88: Stentstrut
- 90: Steg
- 92: Kreuzungspunkt
- 94: Verbindungsstelle
- 96: Ventrikel
- 98: Kalzifizierung
- 100: Implantationsrichtung

## Patentansprüche

1. Medizinisches Klappenimplantat (12), zur Implantation im tierischen und/oder menschlichen Körper (14) mit einem wenigstens bereichsweise expandierbaren Grundkörper (16), der wenigstens bereichsweise expandierbare Grundkörper (16) einen ersten aktiv expandierbaren Bereich (18) und zumindest einen zweiten passiv expandierbaren Bereich (20, 22) aufweist, wobei der Grundkörper (16) zumindest zwei zweite Bereiche (20, 22) aufweist, **dadurch gekennzeichnet** das der Grundkörper eine Klappe (52) aufweist, die in einer axialen Richtung (26) zumindest zwischen zwei zweiten Bereichen (20, 22) angeordnet ist und zumindest ein zweiter Bereich in radialer Richtung weiter außen angeordnet ist als der erste Bereich.

2. Medizinisches Implantat nach Anspruch 1, **dadurch gekennzeichnet, dass** zumindest zwei zweite Bereiche weiter außen angeordnet sind als der erste Bereich und/oder zwei zweite Bereich über dem ersten Bereich angeordnet sind.

3. Medizinisches Implantat nach Anspruch 1 oder 2, **dadurch gekennzeichnet, dass** der zweite Bereich (20, 22) zeitlich nach dem ersten Bereich (18) expandierbar ist.

4. Medizinisches Implantat nach einem der vorangegangenen Ansprüche, **dadurch gekennzeichnet, dass** der erste Bereich (18) dazu vorgesehen ist, bei einer Expansion zumindest des ersten Bereichs (18) den zweiten Bereich (20, 22) in einer transversalen Richtung (24) aufzuweiten.

5. Medizinisches Implantat nach einem der vorhergehenden Ansprüche, **dadurch gekennzeichnet, dass** der zweite Bereich (20, 22) mit dem ersten Bereich (18) über einen sich in einer axialen Richtung (26) erstreckenden Bereich (28) verflochten ist.

6. Medizinisches Implantat nach einem der vorhergehenden Ansprüche, **dadurch gekennzeichnet, dass** der Grundkörper (16) mittels einer Expansion in seiner axialen Länge (30) verkürzbar ist.

7. Medizinisches Implantat nach einem der vorhergehenden Ansprüche, **dadurch gekennzeichnet, dass** der zweite Bereich (20, 22) dazu vorgesehen ist, einen Unterschied in einer Form eines Innenquerschnitts (32) des Grundkörpers (16) und einer Querschnittsfläche (34) einer Implantationsstelle (36) auszugleichen.

8. Medizinisches Implantat nach einem der vorhergehenden Ansprüche, **gekennzeichnet durch** ein Trennmittel, (38) das den ersten Bereich (18) von dem zweiten Bereich (20, 22) trennt.

9. Medizinisches Implantat nach einem der vorhergehenden Ansprüche, **gekennzeichnet durch** zumindest ein Verankerungsmittel (40) zumindest des zweiten Bereichs (20, 22), das im bestimmungsgemäßen Endzustand zumindest einen Extremalpunkt (42) aufweist, der ausgehend von einem geometrischen Schwerpunkt (44) einer von dem ersten Bereich (18) aufgespannten Fläche (46) einen größeren Abstand (48) zu dem Schwerpunkt (44) aufweist als ein Abstand (50) des ersten Bereichs (18) zu dem Schwerpunkt (44).

10. Medizinisches Implantat nach einem der vorhergehenden Ansprüche, **dadurch gekennzeichnet, dass** die zumindest zwei zweiten Bereiche (20, 22) im bestimmungsgemäßen Endzustand in Fließrichtung (54) eines Fließmediums (56) axial vor und hinter einem Annulus (58) angeordnet sind.

11. Medizinisches Implantat nach einem der vorhergehenden Ansprüche, **dadurch gekennzeichnet, dass** der zweite Bereich (20, 22) des Grundkörpers (16) zumindest Kobalt und/oder Chrom aufweist.

12. Medizinisches Implantat nach einem der vorhergehenden Ansprüche, **dadurch gekennzeichnet, dass** der Grundkörper (16) einen Stent (60) umfasst.

13. Medizinisches Implantat nach einem der vorhergehenden Ansprüche, **dadurch gekennzeichnet, dass** das Trennmittel amorphes Siliziumcarbid umfasst.

14. Medizinisches Implantat nach einem der vorhergehenden Ansprüche, **gekennzeichnet durch** eine Ausgestaltung als Aortenklappe (62).

## Claims

1. A medical valve implant (12) for implantation in an animal and/or human body (14), comprising a main body (16) that can be expanded at least in some regions, said main body (16) that can be expanded in at least some regions having a first actively expandable region (18) and at least one second passively expandable region (20, 22), wherein the main body (16) has at least two second regions (20, 22), **characterised in that** the main body has a valve (52), which is arranged in an axial direction (26) at least between two second regions (20, 22) and at least one second region is disposed further to the outside in the radial direction than the first region.

2. The medical implant according to Claim 1, **characterised in that** at least two second regions are disposed further to the outside than the first region and/or two second regions are arranged above the first region.

3. The medical implant according to Claim 1 or 2, **characterised in that** the second region (20, 22) can be expanded temporally after the first region (18).

4. The medical implant according to one of the preceding claims, **characterised in that** the first region (18) is provided to widen the second region (20, 22) in a transversal direction (24) upon an expansion of at least the first region (18).

5. The medical implant according to one of the preceding claims, **characterised in that** the second region (20, 22) is intertwined with the first region (18) over a region (28) extending in an axial direction (26).

6. The medical implant according to one of the preceding claims, **characterised in that** the main body (16) can be shortened in the axial length (30) thereof by way of an expansion.

7. The medical implant according to one of the preceding claims, **characterised in that** the second region (20, 22) is provided to compensate for a difference between a shape of an inside cross section (32) of the main body (16) and a cross-sectional area (34) of an implantation site (36).

8. The medical implant according to one of the preceding claims, **characterised by** a separating means (38), which separates the first region (18) from the second region (20, 22).

9. The medical implant according to one of the preceding claims, **characterised by** at least one anchoring means (40) of at least the second region (20, 22), which in an intended final state has at least one extremal point (42), which starting from a geometric centre of gravity (44) of an area (46) spanned by the first region (18) has a larger distance (48) from the centre of gravity (44) than a distance (50) of the first region (18) from the centre of gravity (44).

10. The medical implant according to one of the preceding claims, **characterised in that** in an intended final state the at least two second regions (20, 22) are disposed axially in front of and after an annulus (58) in the flow direction (54) of a flow medium (56).

11. The medical implant according to one of the preceding claims, **characterised in that** the second region (20, 22) of the main body (16) comprises at least cobalt and/or chromium.

12. The medical implant according to one of the preceding claims, **characterised in that** the main body (16) comprises a stent (60).

13. The medical implant according to one of the preceding claims, **characterised in that** the separating means comprises amorphous silicon carbide.

14. The medical implant according to one of the preceding claims, **characterised by** an embodiment as an aortic valve (62).

## Revendications

1. Implant médical de valvule (12) pour une implantation dans le corps animal et/ou humain (14) avec un corps de base (16) pouvant être en expansion au moins localement, le corps de base (16) pouvant être en expansion au moins localement présente une première partie active en expansion (18) et au minimum une deuxième partie passive en expansion (20, 22), où le corps de base (16) présente au minimum deux deuxièmes parties (20, 22), **caractérisé en ce que** le corps de base présente une valve (52) qui est disposée dans une direction axiale (26) au minimum entre deux deuxièmes parties (20, 22) et au minimum une deuxième partie est disposée dans la direction radiale plus loin à l'extérieur que la première partie.

2. Implant médical selon la revendication 1, **caractérisé en ce qu**'au minimum deux deuxièmes parties sont disposées plus loin à l'extérieur que la première partie et/ou deux deuxièmes parties sont disposées au dessus de la première partie.

3. Implant médical selon les revendications 1 ou 2, **caractérisé en ce que** la deuxième partie (20, 22) peut être mise en expansion de façon décalée dans le temps après la première partie (18).

4. Implant médical selon l'une des revendications précédentes, **caractérisé en ce que** la première partie (18) est prévue pour élargir la deuxième partie (20, 22) dans une direction transversale (24) lors d'une expansion d'au minimum la première partie (18).

5. Implant médical selon l'une des revendications précédentes, **caractérisé en ce que** la deuxième partie (20, 22) est tressée avec la première partie (18) par l'intermédiaire d'une partie (28) s'étendant dans une direction axiale (26).

6. Implant médical selon l'une des revendications précédentes, **caractérisé en ce que** le corps de base (16) peut être raccourci au moyen d'une expansion dans sa longueur axiale (30).

7. Implant médical selon l'une des revendications précédentes, **caractérisé en ce que** la deuxième partie (20, 22) est prévue pour compenser une différence dans une forme d'une section transversale intérieure (32) du corps de base (16) et une surface d'une section transversale (34) d'un point d'implantation (36).

8. Implant médical selon l'une des revendications précédentes, **caractérisé par** un produit de séparation (38) qui sépare la première partie (18) de la deuxième partie (20, 22).

9. Implant médical selon l'une des revendications précédentes, **caractérisé par** au minimum un moyen d'ancrage (40) de l'au moins deuxième partie (20, 22) qui présente, dans l'état final conforme, au moins un point extrême (42), qui présente une distance (48) partant du centre de gravité géométrique (44) d'une surface (46) sous-tendue à la première partie (18) jusqu'au centre de gravité (44) supérieure à une distance (50) de la première partie (18) jusqu'au centre de gravité (44).

10. Implant médical selon l'une des revendications précédentes, **caractérisé en ce que** les au moins deux deuxièmes parties (20, 22) sont disposées axialement dans la direction d'écoulement (54) d'un milieu d'écoulement (56) avant et après un anneau (58) à l'état final conforme.

11. Implant médical selon l'une des revendications précédentes, **caractérisé en ce que** la deuxième partie (20, 22) du corps de base (16) présente au moins du cobalt et/ou du chrome.

12. Implant médical selon l'une des revendications précédentes, **caractérisé en ce que** le corps de base (16) comprend un stent (60).

13. Implant médical selon l'une des revendications précédentes, **caractérisé en ce que** le produit de séparation comprend du carbure de silicium amorphe.

14. Implant médical selon l'une des revendications précédentes, **caractérisé par** une conception sous forme de valvule aortique (62).
